# EUROPEAN PATENT APPLICATION

(11) **EP 0 566 816 A1**
(43) Date of publication of application: **27.10.1993**
(21) Application number: 92850234.3
(22) Date of filing: 07.10.1992
(51) Int. Cl.: A61K 9/70, A61F 13/00, A61L 15/00

(54) **Two-hand pouch patch application**

(30) Priority: 21.04.1992 US 871342
(71) Applicant: MLI ACQUISITION CORP. II, Morgantown, West Virginia 26505-4310 (US)
(72) Inventor: Wick, John, Essex Junction Vermont 05452 (US)
(74) Representative: Mossmark, Anders

(57) **Abstract**

Delivery systems are disclosed including a carrier sheet (13) with a releasable surface, a thin film (7) adhesively and releasably secured to the releasable surface of the carrier sheet, removal means (11) for removing the thin film from the releasable surface of the carrier sheet, and a cover sheet (3) secured to the carrier sheet beyond the periphery of the thin film characterized by the cover sheet (3) being free of adhesive in its entire area in contact with the thin film (7).

## Description

The present invention relates to delivery systems. More particularly, the present invention relates to devices for the handling and delivery of wound dressings and transdermal applicators. Still more particularly, the present invention relates to methods for manufacturing such delivery systems.

There are in existence an extremely large number of skin contact devices, including wound dressing, wound dressing delivery systems, and systems for transdermal application of various drugs, which have been developed over the years. The field of wound dressings includes the application of film layers which can be free of any active agent medicament or which can include such components for transdermal delivery. In general, these devices have been developed over the years to assist in the healing of wounds faster than was previously possible by utilizing films capable of maintaining well-balanced environments with respect to air, moisture, and the like.

One of the most significant recent developments in the field of such medical and surgical dressings is set forth in U.S. Patent No. 4,915,102. The background section of this patent includes a detailed discussion of a number of additional devices developed prior to that date and whose function was to provide various types of wound dressings and the like.

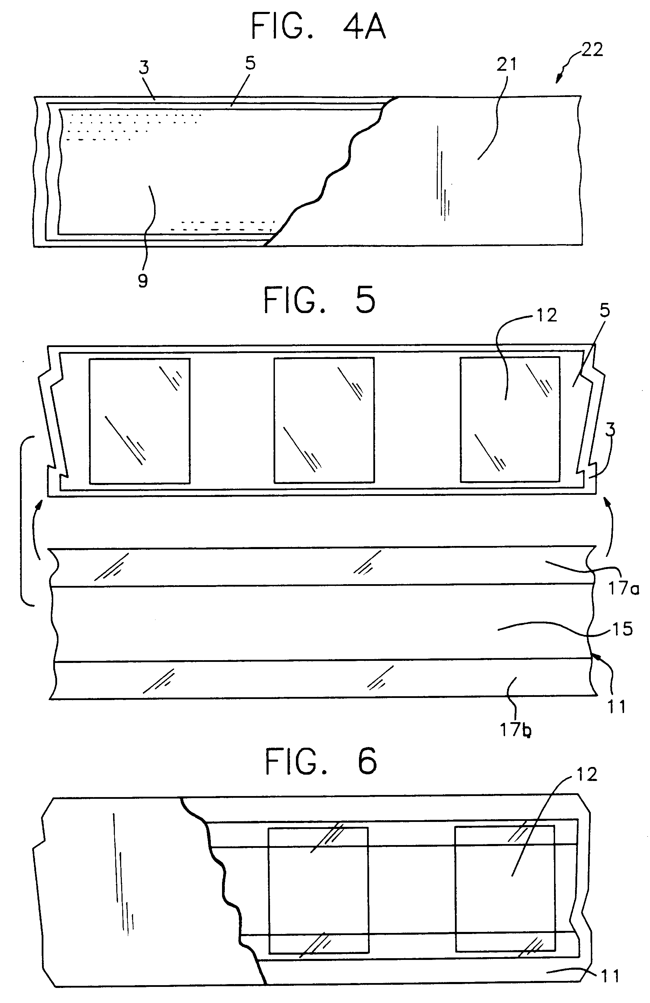

The delivery system which is disclosed in U.S. Patent No. 4,915,102 is particularly significant in that it makes it possible to apply a thin film in a one-hand procedure; i.e., after opening of the delivery device and application to the skin, the carrier layer can be readily removed therefrom. The devices disclosed in this patent thus include a liner layer 6, a carrier layer 4, and a film layer 2 deposited thereon, as shown in FIGS. 5-8 thereof.

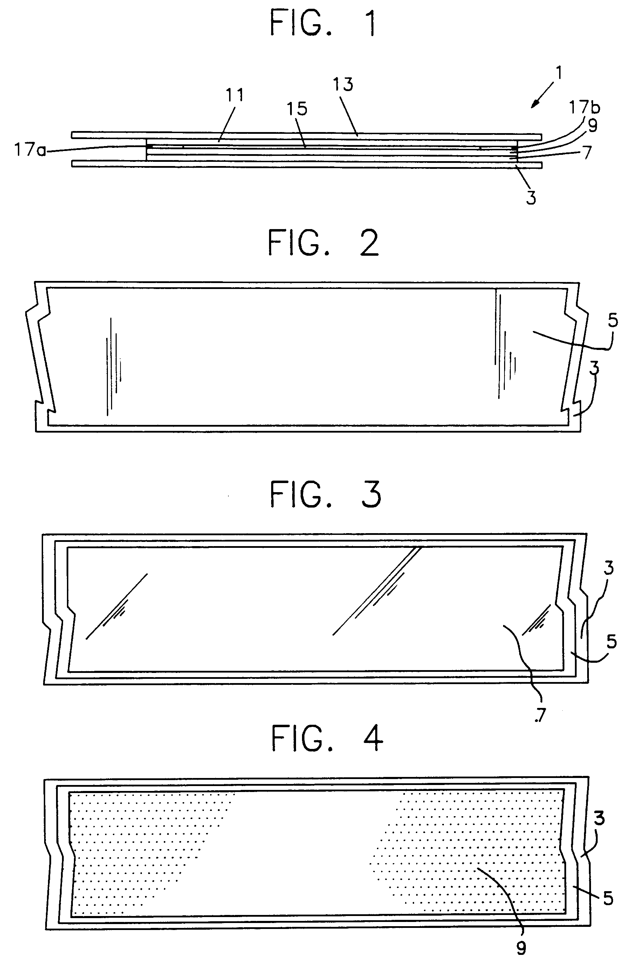

Another wound dressing device is shown in U.S. Patent No. 4,884,563. In this case, the wound dressing includes a two-tab system in one embodiment thereof, as shown in FIG. 3, such that a first tab 12 is pulled causing release of adhesive layer 11 from release surface 9 with cover sheet 15 adhering to the opposite side thereof. After this combination is placed on the wound itself, tab 13 is pulled, thus removing cover sheet 15 and leaving a film 10 adhering to the patient.

Additional wound dressings are shown, for example, in U.S. Patent No. 4,561,435, and U.S. Patent No. 4,499,896, as well as U.S. Patent No. 4,485,809.

Irrespective of the fact that so many different such delivery systems exist, however, the search has continued for new systems for specific applications. For example, the search for a two-hand delivery system, which is simple to manufacture and which renders it possible to provide a self-contained product which is easy to utilize, has continued.

In accordance with the present invention, these and other objects have now been met by the invention of a delivery system which includes a carrier sheet having a releasable surface, a thin film adhesively and releasably secured to the releasable surface of the carrier sheet, removal means for removing the thin film from the releasable surface of the carrier sheet, and a cover sheet secured to the carrier sheet beyond the periphery of the thin film, the cover sheet being free of adhesive in its entire area in contact with the thin film.

In accordance with one embodiment of the delivery systems of the present invention, the removal means includes tab means adhesively secured to a predetermined portion of the thin film, such that the thin film can be removed from the releasable surface of the carrier sheet by gripping the tab means. Preferably, the tab means is freely removed from the releasable surface of the carrier sheet.

In accordance with another embodiment of the delivery system of the present invention, the carrier sheet is heat sealed to the cover sheet about the periphery of the thin film so as to enclose the thin film therewithin.

In accordance with the method of the present invention, a method for manufacturing a delivery system comprises providing a cover sheet having a releasable surface, applying a thin film to the releasable surface of the cover sheet, coating the thin film with an adhesive, providing a removal sheet comprising a first surface and a second surface, the removal sheet including a primary portion and a secondary portion, applying the first surface of the removal sheet to the adhesive-coated thin film, whereby the primary portion of the removal sheet contacts the major portion of the adhesively coated thin film, and a secondary portion of the removal sheet contacts a minor portion of the adhesively coated thin film, covering the adhesively coated thin film with a carrier sheet having a releasable surface thereon, and heat treating the primary portion of the removal sheet whereby the primary portion of the removal sheet becomes adhesively secured to the carrier sheet and the secondary portion of the removal sheet becomes adhesively secured to the thin film.

In accordance with one embodiment of the method of the present invention, the method includes heat sealing the cover sheet to the carrier sheet peripherally beyond the thin film. In another embodiment, application of the thin film to the cover sheet comprises extrusion coating thereof.

The present invention can be more readily understood with reference to the following detailed description, in which reference is made to the drawings wherein:
FIG. 1 is a side, enlarged, cross-sectional view of a delivery system in accordance with the present invention;
FIG. 2 is a top, partial view of a first stage in the method of manufacturing a delivery system in accordance with the present invention;
FIG. 3 is a top, partial view of a second step in the method of manufacturing a delivery system in accordance with the present invention;
FIG. 4 is a top, partial view of a third step in a method for manufacturing a delivery system in accordance with the present invention;
FIG. 4a is a top, partially cut-away view of a fourth step in a method for manufacturing a delivery system in accordance with the present invention;
FIG. 5 is a separated, top, partial view of a fourth step in a method for manufacturing a delivery system in accordance with the present invention;
FIG. 6 is a top view of a fifth step in a method for manufacturing a delivery system in accordance with the present invention;
FIG. 7 is a top view of a sixth step in a method for manufacturing a delivery system in accordance with the present invention;
FIG. 8 is a top, partial view of a seventh step in a method for manufacturing a delivery system in accordance with the present invention.
FIG. 9 is a top view, through a transparent carrier sheet, of an eighth step in a method for manufacturing a delivery system in accordance with the present invention.
FIG. 10 is a schematic representation of an overall method for manufacturing a delivery system in accordance with the present invention; and
FIG. 11 is a side, enlarged, cross-sectional view of another delivery system in accordance with the present invention.

An important element in both the delivery system produced in accordance with the present invention and in the method for manufacturing it relates to the fact that the product itself includes a cover layer which forms an integral part of the product, thus providing a component for sealing the thin film therewithin for packaging purposes, is readily removed from the device to open the package and initiate the application process, and, in addition, acts as a base for the preparation and manufacture of the product itself. Thus, in the final product, although this cover sheet is so integral in producing the product itself, it is free of adhesive in its entire area which contacts the thin film which is to be applied therefrom.

Turning to the Figures, in which like reference numerals refer to like portions thereof, FIG. 1 shows an expanded cross-sectional view of a delivery system in accordance with this invention. Thus, the delivery system 1 includes a cover sheet 3 which is discussed above and in greater detail hereinbelow. The overall delivery system is thus bounded by cover sheet 3 on one side and a carrier sheet 13 on the other. The carrier sheet actually "carries" the thin film 7 so that upon removal of the cover sheet 3 the thin film 7 can ultimately be applied to the user from the carrier sheet 13.

The cover sheet 3 itself must include a releasable surface. Thus, cover sheet 3 can comprise a number of different types of materials, including paper or paper-containing layers or laminates, as well as various thermoplastics, such as extruded polyolefin, such as polyethylene, as well as foil liners, or various laminates of these different layers. However, cover sheet 3 must have an inner surface which is releasable with respect to the materials or layers with which it is initially in contact. It should therefore include a release coating, such as where a paper layer or the like is employed. This can be done in a conventional manner, such as by using a silicon or teflon coating on the surface thereof.

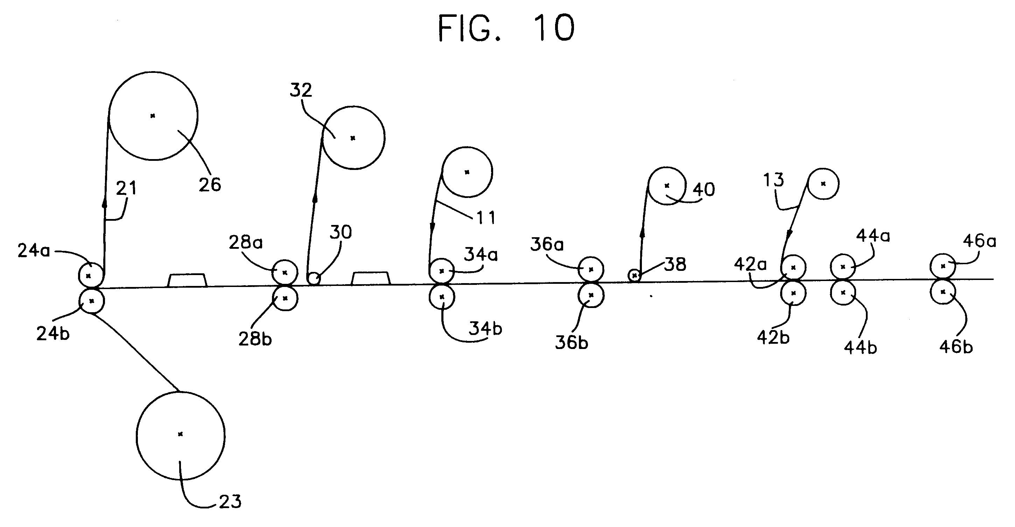

This is particularly shown in FIG. 2, where the cover sheet 3 has a release coating 5 which has been applied thereto, preferably by an extrusion process. This particular aspect of the present invention, as well as the overall method of producing the product shown in FIG. 1, will be discussed below in connection with the method hereof, as shown in FIG. 10.

The thin film 7 generally has a high degree of elasticity and also has a releasable surface. It is desirable for the thin film 7 to have sufficient tensile strength and deformability characteristics to resist becoming torn as when a user attempts to remove it from its assembled position in delivery system 1. Thus, the thin film 7 can comprise a polymer or an elastomer. The releasable surface of thin film 7 is disposed adjacent the release coating 5 of the cover sheet 3, as shown in FIG. 3, thereby providing for easy separation between same.

The thin film 7 generally comprises an extremely thin and flimsy layer of material, which is preferably breathable and non-occlusive in nature. The thin film 7 is a material which would be extremely difficult, if not impossible, to handle by itself, i.e., apart from the removal sheet 11 and carrier sheet 13, which will be described in more detail hereinbelow.

The thin film 7 is intended to act as a wound dressing and is thus a relatively flimsy material, which preferably allows air and moisture vapor to pass therethrough, but which will not permit the passage of bacteria or undesired elements or materials. It is preferred that the thin film 7 be composed of various thin, plastic materials or it can comprise an extremely thin foil layer, or layer of other non-woven materials, most particularly it will comprise a material which is sufficiently thin and flexible to be conformable to the skin, and will thus preferably be thinner than about 2 mils, more preferably less than about 1.5 mils, and the most preferably 1 mil or less.

The thin film 7 preferably comprises a thermoplastic material which is at least partially elastomeric in nature. Thus, it will exhibit a high degree of elongation, preferably greater than 130% elongation, and will also exhibit excellent conformability characteristics without having the tendency to exhibit significant memory characteristics, although it will have some degree of recovery if stretched as during removal from the carrier sheet 13. In terms of being breathable, it is preferred that the thin film 7, in addition to permitting air to pass therethrough, will also permit some amount of moisture vapor to pass therethrough, at least more readily than is the case with non-breathable materials such as polyethylene. The thin film must be occlusive, at least with respect to particulates, in order to protect a particular wound, however, its overall occlusive characteristics can vary, depending upon its ultimate intended use. Preferably, the thin film 7 will be permeable to various glycols, such as polyethylene glycols, however, more occlusive materials can also be employed for the purpose of thin film 7 in selected circumstances. For example, one and two mil layers of ethylene-vinyl acetate copolymers, or various nylon or polyester films may be useful in various applications. Additionally, laminated or coated films could also be utilized, such as employing a non-occlusive film such as those discussed above which is fully or partially selectively coated with an occlusive film.

The thin film 7 can generally be produced with either a metallic, glossy or a clear surface, and it can also be selected to include various colors such as skin color, or may have an opaque appearance by utilizing fillers, such as TiO2, clay or other such materials. The thin film 7 can also comprise various organic additives, odor inhibitors, and/or various medications, etc.

From the commercial viewpoint, one of the most successful high moisture, vapor permeable, medical grade elastomeric films, suitable to fulfill the purposes of the thin film 7, has been one of a series of products marketed by DuPont under the designation "Medifilm 800." These medical grade elastomeric films are extruded from a class of elastomeric resins which are polyether block amides, commercially designated by the trademark PEBAX. The structure of these polymers can be generally represented by the formula: 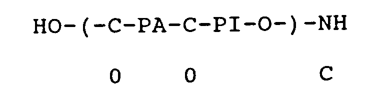
in which PA represents a relatively rigid polyamide segment and PE represents a relatively soft polyether segment. In this manner the extruded film products have high strengths in terms of high tear and abrasion resistance and at the same time provide a high degree of comfort or conformability, as well as moisture vapor permeability. The physical properties of two typical medical grade PEBAX films having a thickness of 1 mil are set forth in TABLE I herein.

**TABLE I**

| | FILMS | |
|---|---|---|
| | MEDIFILM | MEDIFILM |
| PROPERTIES | 810 | 827 |
| Tensile strength -psi (ASTM D882) | 3120 | 2200 |
| % Elongation | 430 | 800 |
| Modulus @ 50% elongation | 1600 | 900 |
| Initial tear resistance -lbs. (ASTM D-1004) | 0.65 | 0.6 |
| MUTR -g/m2/24 HRS. (ASTM E-96) 37.8 C/90% R.H. | 1675 | 2200 |

In addition, other acceptable materials to be employed as the thin film 7 can comprise thermoplastic polyurethanes, which also meet the above requirements. These materials include such commercial polyurethane compositions as Dow Chemical Company's PELLETHANE, including its 2363-80 AE grade thereof; K. J. Quinn's Q-THANE; B. F. Goodrich's ESTANE; Mobay Chemical Company's TXIN; and others. Furthermore, the thin film 7 can also comprise various polyesters, such as the copolymers of various cycric polyesters including Dupont's HYTRELL, including its 4056 grade thereof, and General Electric's LOMOD, both of which are copolymers of polyether prepolymers and polybutylene tereththalate and polyisobutyl terephthalate, respectively as well as Eastman Chemical's PCCE.

As illustrated in FIG. 4, an adhesive layer 9 is then applied to a second surface on thin film 7 remote from cover sheet 3. As can be appreciated, adhesive layer 9 can comprise a number of different suitable adhesives, including epoxies, which are particularly desirable since they readily adhere to smooth surfaces such as that on thin film 7. It is preferable for the adhesive layer 9 to comprise an adhesive which is strong enough to provide a suitable bond between the thin film 7 and a removal sheet 11, which will be described in more detail hereinbelow, yet weak enough to enable a person to easily break the bond when it is desired to apply the medication of delivery system 1 to a patient. In another preferred embodiment of the present invention, it is unnecessary to include adhesive layer 9, as thin film 7 can comprise a material which can be suitably heat sealed to an enjoining surface, such as the removal sheet 11.

As for the properties and characteristics of the adhesive to be used in this preferred embodiment of the present invention, it is desirable for the adhesive to be hypoallergenic since the thin film 7 is preferably being applied to the skin of a patient. When the delivery system 1 is used in connection with the application of various drug delivery systems, adhesive layer 9 will then have other more stringent requirements, such as its compatibility with the drug or drugs in question, as well as its resistance to alcohol or other solvents utilized thereon, etc. In connection with the application of adhesive layer 9 on the thin film 7 above, an acrylic copolymer adhesive such as Avery Chemical Company's AS-351 HSX can be utilized, preferably at a coating weight of between about 25 and 35 g/ms2. This pressure-sensitive adhesive is a cross-linkable polymer which dries to provide a permanently tacky film having a total solids content of about 52%, a Brookfield viscosity (LVT/04/12 RPM @ 25'C) of from about 15,000 to 25,000 cps and a weight per gallon of about 7.4 lbs. It can also be diluted with hexane or toluene to a desired solids and/or viscosity range, particularly for use in conventional coating equipment. Other such adhesives, which are particularly suitable for use as the adhesive layer 9 to be used in connection with drug delivery systems, include an acrylic pressure-sensitive adhesive sold by National Adhesives under the designation DUROTAK 80-1054. This acrylic adhesive has a solids content of 47.5%, a viscosity of 3,000 cps, and a plasticity (Williams) of 2.9 mm. It is generally used in connection with a solvent system including ethyl acetate, heptane, isopropyl alcohol and toluene. Another suitable adhesive is sold by Monsanto under the designation GELVA Multipolymer Emulsion 2484, which comprises a stable aqueous acrylic emulsion pressure-sensitive adhesive having a solids content of 59% and a viscosity of 1500-Z, 300 cps. With the above alternate adhesives, the adhesion strength can be reduced by adding additional cross-linking additives thereto and/or by utilizing different coating weights and/or viscosities therefore.

In a preferred embodiment, the removal sheet 11 is positionable adjacent the thin film 7, separated only by adhesive layer 9. The removal sheet 11 facilitates removal of the thin film 7 from the carrier sheet 13 for timely application to a patient. Thus, a portion of the removal sheet 11 also includes a release coating comprising any suitable material such as silicon or TEFLON, disposed for direct contact with the adhesive layer 9 on the thin film 7. This release coated portion of the removal sheet 11 encompasses an area disposed for alignment with a major portion of the thin film 7 beneath the adhesive layer 9. The removal sheet 11 also includes a non-release coated portion which is alignable with a minor portion of the thin film 7 to allow the adhesive layer 9 to form a bond therebetween. Accordingly, the bond formed between the non-release coated portion of the removal sheet 11 and the adhesive layer 9 on the minor portion of the thin film 7, is of a much greater tensile strength than the bond formed between the release coated portion of the removal sheet 11 and the major portion of the thin film 7. Preferably, the removal sheet 11 is separated into two separate portions between the release coated area and the non-release coated area, either before or after it has been adhesively connected to the thin film 7. Thus, the non-release coated area of the removal sheet 11 becomes permanently sealed to the minor portion of the thin film 7 and effectively serves as a tab which provides a means for removing the thin film 7 from the release coated portion of the removal sheet 11, upon gripping of the tab and pulling the tab in a direction toward the opposite end of the thin film 7.

In another preferred embodiment, the removal sheet 11 may include two non-release coated portions disposed along opposite ends, separated by a centrally disposed release-coated portion. The non-release coated portions of the removal sheet 11 are alignable with opposite ends of the thin film 7 to form a permanent bond therebetween. Similar to the above embodiment, the bond formed between the thin film 7 and the non-release coated portions are relatively permanent in comparison to the bond formed between the centrally disposed release coated portion of the removal sheet 11 and the thin film 7. Thus, in this preferred embodiment two permanently bonded tabs are formed on opposite ends of the thin film 7 thereby providing the user of delivery system 1 with the option of removing the thin film 7 from the removal sheet 11 upon gripping either of the two permanently secured gripping tabs and pulling same toward the opposite end of the thin film 7 as described above.

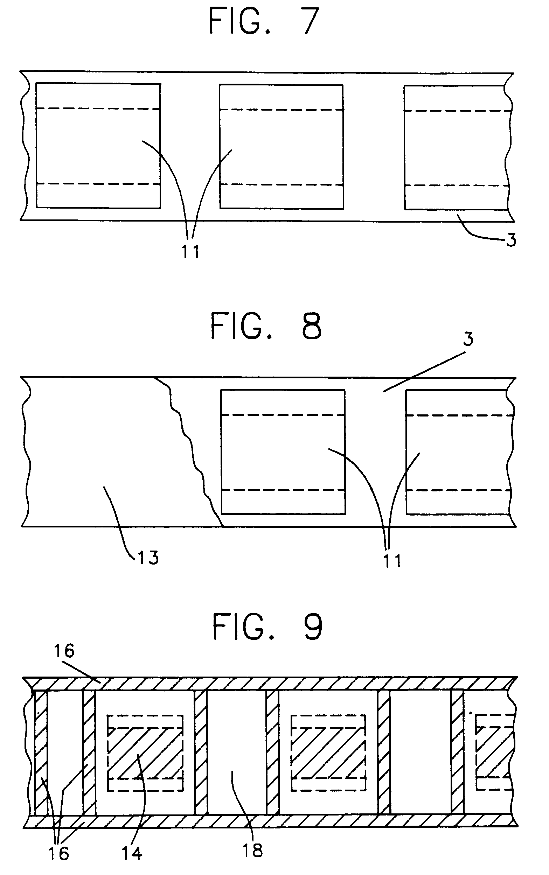

The removal sheet 11 has a second surface remote from the surface adjacent the thin film 7. The second surface is generally non-release coated and when delivery system 1 is in its assembled form, the second surface of the removal sheet 11 is disposed immediately adjacent a carrier sheet 13 as evident from the partial top plan view shown in FIG. 8.

Thus, the removal sheet 11 generally comprises various layers selected for facilitation of its intended purposes, which in a preferred embodiment as described above, comprises protection of the thin film 7 and facilitation of its removal therefrom, and support and protection of the adjacent carrier sheet 13. Accordingly, the various layers of the removal sheet 11 can include paper or paper-containing layers or laminates; various thermoplastics, such as extruded polyolefins, such as polyethylene; foil liners; or various laminates of these different layers. Such materials are also suitable to meet the intended purpose of the carrier sheet 13, which will be described in more detail hereinbelow. However, as discussed above, the removal sheet 11 must have a release coated surface which is releasable with respect to the adhesive layer 9, the drug composition or other materials with which it is disposed for contact with. Thus, where the removal sheet 11 comprises a paper layer or the like, a suitable material such as silicon or TEFLON can be applied to the paper layer or other composition in any conventional manner suitable for such coating operations. Additionally, the removal sheet 11 may include other suitable layers such as fabric layers, coated or laminated to various polymers, as well as extruded polyethylene, polyethylene terephthalate, various polyamides, and the like. The selection of a particular removal sheet 11 will also depend upon other ultimate requirements of the particular device in question, including whether there is a desire for a transparent or opaque removal sheet, etc. Moreover, selection of the removal sheet 11 may depend upon the desired method of bonding the non-release coated second surface, remote from the surface adjacent the thin film 7, to the adjacent carrier sheet 13 as discussed above. Thus, whether the non-release coated surface of the removal sheet 11 is adhesively bonded to the carrier sheet 13, or whether it is sealed thereto, is a matter of design choice which may necessarily effect the choice of a suitable composition for at least the layer comprising the non-release coated surface of the removal sheet 11.

Returning to FIG. 1, the carrier sheet 13, which has the function of "carrying" the thin film of the invention prior to its application to a patient's skin, should basically be composed of a material which is flexible, but which has sufficient substance and strength so that it can readily carry the thin film 7 thereon. The carrier sheet 13 should thus have sufficient strength and substance to carry the film layer without wrinkling, etc. The actual material from which the carrier sheet 13 is formed or can be produced can thus include a variety of different materials. Some suitable materials for this layer include, for example, polyethylene, polypropylene, polyvinylidene chloride, polyethylene terephthalate, polyesters, polyamides, and others, as well as laminates of two or more of these layers with each other or with additional layers, such as foil, paper, various fabrics, etc., but in these cases preferably with the polymer layer on the inside, i.e., in contact with and thereby carrying the thin film 7.

As can also be seen from FIG. 1, the outer edges of the carrier sheet 13, as represented by reference numerals 17a and 17b, are release coated in the same manner as is the case with the entire surface of cover sheet 3 discussed above. Thus, these portions of the carrier sheet 13 are extrusion coated with a layer of silicon or TEFLON or other such material acting as a release coating, so that these ends 17a and 17b of the carrier sheet 13 are releasable with respect to a layer, such as the thin film 7 ultimately placed thereon. However, the central portion of the carrier sheet 13, as indicated by reference numeral 15, does not include such a release coating, and this reference numeral is merely included to indicate this area where no such coating is to be applied, i.e., the non-release-coated surface of carrier sheet 13 is exposed between portions 17a and 17b at the ends thereof.

In a preferred embodiment, the non-release coated central portion 15 of the carrier sheet 13 is positioned for alignment with the non-release coated portion of the second side of removal sheet 11. After the carrier sheet 13 is placed on top of the removal sheet 11 and the alignment between the non-release coated portion of the removal sheet 11 and the non-release coated portion 15 of the carrier sheet 13 is completed, a bond between those adjacent surfaces is sufficiently created by heat sealing the same in a conventional manner. In accordance with this preferred embodiment, it is preferred that the outer perimeter of all sides of the cover sheet 3 extends beyond the outer perimeter of all sides of the thin film 7 in order to effectively complete manufacture of the delivery system 1. The outer perimeter of all sides of the carrier sheet 13 also extends beyond the outer perimeter of all sides of the thin film 7 by a sufficient distance to assure that the outer perimeter of the carrier sheet 13 is approximately the same size as the outer perimeter of all sides of the cover sheet 3. Thus, the carrier sheet 13 can be effectively heat sealed to the cover sheet 3 peripherally beyond the thin film 7. As can be appreciated, in another preferred embodiment of the present invention, the carrier sheet 13 can be adhesively secured to the cover sheet 3 peripherally beyond the thin film 7 by the use of any suitable adhesive, such as an epoxy or the like.

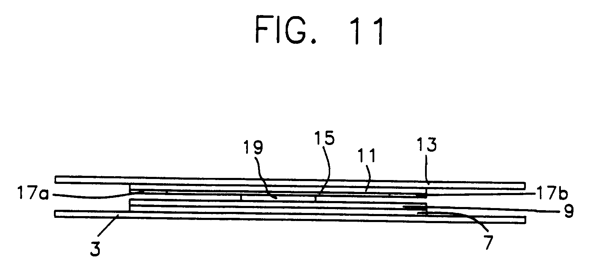

Referring now to FIG 11, another preferred embodiment of the present invention is illustrated wherein an absorbent sheet 19 having a first and a second side is disposed with its first side immediately adjacent the adhesive layer 9 on the thin film 7 and its second side immediately adjacent removal sheet 11. Most preferably, the second side of the absorbent sheet 19 comprises a drug composition. The drug composition can vary widely depending on its intended use and application. Depending upon the heat sensitivity of the drug composition, the choice of the drug composition will influence the bonding method between the removal sheet 11 and the carrier sheet 13. Beyond the sensitivities of the drug composition, the choice of heat sealing or adhesively attaching the removal sheet 11 to the carrier sheet 13 and the periphery of carrier sheet 13 to the periphery of cover sheet 3, is merely a choice of manufacture and can be effectuated in any conventional manner as discussed above.

It is desirable in this preferred embodiment for the absorbent sheet 19 to comprise various layers, one of which is generally non-absorbent and is disposed immediately adjacent the adhesive layer 9 on the thin film 7 as described above. This relatively non-absorbent layer of the absorbent sheet 19 is preferably composed of a thermoplastic material being at least partially elastomeric in nature, as is the thin film 7. Preferably, this relatively non-absorbent layer of absorbent sheet 19 will comprise a material which is sufficiently flexible to further the purpose of the overall delivery system 1. Various such non-absorbent materials will be apparent to those of ordinary skill in this art.

As for the second, relatively absorbent, layer of the absorbent sheet 19, it will be disposed in assembled form to be in direct contact with the drug or drugs to be used in connection with the delivery system 1. Thus, the selection of a suitable composition for this relatively absorbent layer of absorbing sheet 19 is critical, as its compatibility with the drug or drugs in question must be carefully considered. This layer of the absorbent sheet can be chosen from a wide selection of commercially practical materials, which have already been subjected to stringent compatibility tests with corresponding drug compositions, such as the various absorbent materials which are conventionally utilized as wound dressings and the like. These absorbent sheets 19 can thus include woven gauze materials, such as REEMAX, a trademark of Johnson & Johnson Company; cotton pads; various forms of hydrogels; and, as noted above, can include various active ingredients or medicaments thereon, such as those set forth at column 10, line 27, through column 12, line 13 of U.S. Patent No. 4,573,996, the disclosure of which is incorporated herein by reference thereto. In addition, both absorbent materials themselves (see column 11, lines 54-60) and medicaments and other such components for use therein (see column 11, lines 1-32) are set forth in U.S. Patent No. 4,884,563, the disclosure of which is incorporated herein by reference thereto.

A preferred method for manufacturing the delivery system 1 in accordance with the present invention, is schematically represented in FIG. 10. An important first step in the method of the present invention includes providing a cover sheet which will eventually form an integral part of the delivery system 1, and which will act as a base during manufacture of the present invention. Accordingly, during the manufacture of the present invention, a web comprising the release coated cover sheet, thin film 7 and adhesive layer 9 (See Fig. 4), with a temporary release liner 21 thereon, as shown in Fig. 4A, is supplied by feed roller 23, and is then fed through press rollers 24a and 24b wherein it is pressed to remove any wrinkles that may exist, and in which the temporary release liner 21 is removed by upper roller 24a and collected upon liner roller 26. The combination of thin film 7 and adhesive layer 9 is then divided by means of die cutting rolls 28a and 28b into a plurality of rectangular thin film portions surrounded by excess thin film material. Thereafter, the excess thin film is removed from the web, comprising the cover sheet, by means of excess thin film removal roll 30, and is transported to excess storage roll 32. Significantly, the bottom surface of the thin film portions 12 immediately adjacent the continuous cover sheet remains completely free of any adhesive means or other binding agent during this process.

The next step in this process is illustrated in Fig. 5. A web comprising the removal sheet 11 is fed by means of removal sheet roll 13 through laminating rollers 34a and 34b where it is laminated onto the surface of the cover sheet 3 with the die cut thin film portion 12 thereon. The surface of the removal sheet 11 is positioned to effectively cover the entire surface of the die cut thin film portions 12, and extends beyond the peripheral areas of the die cut thin sheet portion 12, and is disposed to be adhesively bonded thereto.

The removal sheet 11 is itself provided with a primary portion and a secondary portion. As indicated in the foregoing description, the surface of the secondary portion of the removal sheet, which in the example shown in Fig. 5 comprises the two side portions 17a and 17b thereof, are non-release coated, and are therefore suitable for forming a relatively permanent bond with respect to an end of the die cut thin film portions 12, thereby forming a tab sufficient to effectuate later removal of the thin film from the release coated primary portion of the removal sheet between the two secondary portions 17a and 17b. At this stage in the preferred process, the superimposed product of Fig. 6 is present. Then, via die cutting rollers 36a and 36b, the removal sheet 11 is cut to cover only the thin film portions 12 as shown in Fig. 7. The excess portions of the removal sheet are then removed ontoexcess storage roll 40.

The carrier sheet 13 is then applied to the non-release coated surface of the now die cut portions of removal sheet 11 to get the product shown in Fig. 9. This product is fed between the carrier sheet laminating rollers 42a and 42b, and is then heat sealed in the shaded portions shown in Fig. 9 by feeding the product between heat seal rollers 44a and 44b. Finally, the individual delivery systems 1 are cut by final cutting rollers 46a and 46b to place the delivery system in its final form.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A delivery system comprising a carrier sheet (83) including a releasable surface, a thin film (7) adhesively and releasably secured to said releasable surface of said carrier sheet, removal means (11) for removing said thin film from said releasable surface of further comprising said carrier sheet, and a cover sheet (3) secured to said carrier sheet beyond the periphery of said thin film, characterized by said cover sheet (3) being free of adhesive in its entire area in contact with said thin film (7).

2. The delivery system according to claim 1 characterized in that said removal means (11) comprises tab means (17a, 17b) adhesively secured to a predetermined portion of said thin film (7) whereby said thin film can be removed from said releasable surface of said carrier sheet by gripping and pulling said tab means (17a, 17b).

3. The delivery system according to claim 2 characterized in that said tab means (17a, 17b) is freely removed from said releasable surface of said carrier sheet (3).

4. The delivery system according to claim 1, 2 or 3 further characterized by absorbent means (9) adhesively secured to said adhesively coated surface of said thin film (7).

5. The delivery system according to claim 4 characterized in that said absorbent means (19) includes a drug composition.

6. The delivery system according to claim 1, 2 or 3 characterized in that said carrier sheet (13) comprises a base sheet including an extruded film thereon.

7. The delivery system according to claim 1, 2 or 3 characterized in that said carrier sheet (13) is heat sealed to said cover sheet (3) about the periphery of said thin film (7) so as to enclose said thin film (7) therewithin.

8. A method for manufacturing a delivery system comprising providing a cover sheet (3) having a releasable surface, applying a thin film (7) having a major portion and a minor portion to said releasable surface of said cover sheet, coating said thin film with an adhesive layer (9), providing a removal sheet (11) comprising a first surface and a second surface, said removal sheet including a primary portion and a secondary portion, further characterized by said primary portion (19) comprising a release coated surface and said secondary portion (17a, 17b) comprising a non-release coated surface, applying said first surface of said removal sheet (11) to said adhesive layer (9) coated thin film (7), whereby said primary portion of said removal sheet contacts the major portion of said adhesively coated thin film and said secondary portion of said removal sheet contacts a minor portion of said adhesively coated thin film, covering said adhesively coated thin film with a carrier sheet (13) having a releasable surface thereon, and heat treating said primary portion of said removal sheet whereby said primary portion of said removal sheet becomes adhesively secured to said carrier sheet and said secondary portion of said removal sheet remains adhesively secured to said thin film.

9. The method according to claim 8 further characterized by heat sealing said cover sheet (3) to said carrier sheet (13) peripherally beyond said thin film (7).

10. The method according to claim 8 characterized in that said applying of said thin film (7) to said cover sheet (3) comprises extrusion coating thereof.

11. The method according to claim 8 further characterized by covering said adhesively coated (9) thin film (7) with a temporary release liner (21), and removing said temporary release liner prior to applying said first surface of said removal sheet (11) to said adhesively coated (9) thin film (7).

12. The method according to claim 8, 9 or 10 further characterized by dividing said thin film (7) into a plurality of thin film portions (12) prior to applying said removal sheet (11) to said adhesively coated thin film (12).

13. The method according to claim 8, 9 or 10 further characterized by applying an absorbent layer (19) to said adhesively coated thin film (7) prior to applying said removal layer (11).

14. The method according to claim 8, 9 or 10 further characterized by applying an active agent carrier (19) to said adhesively coated thin film (7) prior to applying said removal layer (11).
